# EUROPEAN PATENT SPECIFICATION

(11) **EP 4 345 093 B1**
(45) Date of publication and mention of the grant of the patent: **14.05.2025**
(21) Application number: 23163357.9
(22) Date of filing: 22.03.2023
(51) Int. Cl.: C07C 269/06, C07C 271/22, B01J 19/00

(54) **A PROCESS METHOD FOR SYNTHESIZING QUINOLONES INTERMEDIATES BY USE OF A MICROREACTOR**
VERFAHREN ZUR SYNTHESE VON CHINOLON-ZWISCHENPRODUKTEN UNTER VERWENDUNG EINES MIKROREAKTORS
PROCÉDÉ DE SYNTHÈSE D'INTERMÉDIAIRES DE QUINOLONES AU MOYEN D'UN MICRORÉACTEUR

(30) Priority: 06.09.2022 CN 202211086060
(43) Date of publication of application: 03.04.2024
(73) Proprietor: ZheJiang Medicine Co., Ltd. Xinchang Pharmaceutical Factory, Xinchang, Zhejiang 312500 (CN); Zhejiang Changhai Pharmaceutical Co., Ltd., Shaoxing City, Zhejiang Province 312366 (CN)
(72) Inventor: SHENG, Li, Xinchang, 312500 (CN); LUO, Yuquan, Xinchang, 312500 (CN); FAN, Gang, Xinchang, 312500 (CN); CHEN, Long, Xinchang, 312500 (CN); CHEN, Junwei, Xinchang, 312500 (CN); LV, Chunlei, Xinchang, 312500 (CN); WU, Guofeng, Xinchang, 312500 (CN); SHEN, Dadong, Xinchang, 312500 (CN); ZHAO, Lin, Xinchang, 312500 (CN); GONG, Yunxia, Xinchang, 312500 (CN)
(74) Representative: Fuchs Patentanwälte Partnerschaft mbB

(56) References cited:
- US-A1- 2010 152 452

## Description

### TECHNICAL FIELD

The present disclosure relates to the field of organic compound synthesis, in particular to a process method for synthesizing quinolones intermediates by use of a microreactor.

### BACKGROUND

Nemonoxacin malate has a structural formula as shown in formula v, and a chemical na me of 7-[(3S, 5S)-3-amino-5-methyl-piperidin-1-yl]-1-cyclopropyl-8-methoxy-4-oxo-1,4-dihydroqui noline-3-carboxylate malate hemihydrate. It is the world's first fluoroquinolone-free antibacteria I drug developed by Procter & Gamble, US., and Phase II clinical studies on community pn eumonia and diabetic foot infection have been completed in the United States, with significa nt effects. Phase III clinical trials of community pneumonia with oral dosage form jointly con ducted by Chinese Mainland and Taiwan have also been completed, and marketed in 2016.

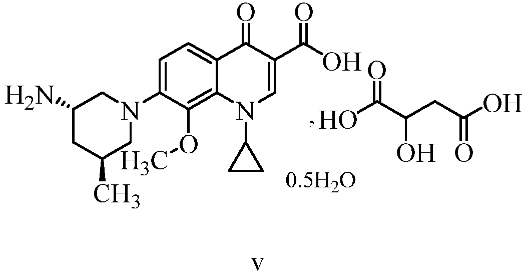

Nemonoxacin malate of formula v is prepared by salifying the intermediate Nemonoxacin as shown in formula iv. The intermediate Nemonoxacin vi is generally prepared by condensation and hydrolysis of 1-cyclopropyl-7-fluoro-8-methoxy-4-oxo-1,4-dihydro-quinolin-3-carboxylic acid diacetate boric anhydride iii and piperidine derivative iv in the presence of an acid binding agent, and the synthesis route is as shown in reaction formula I: 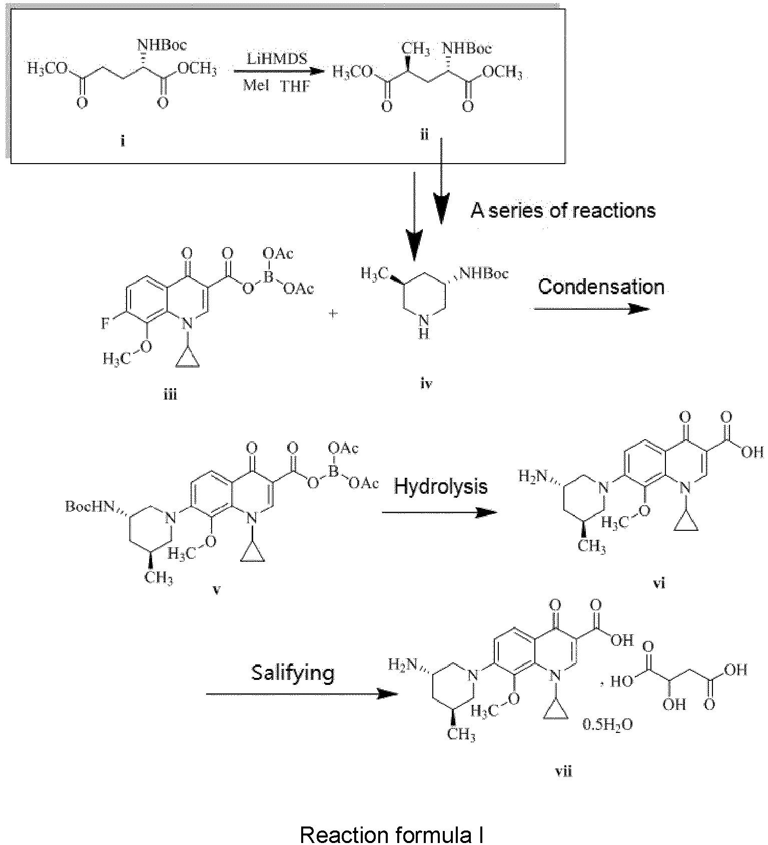 wherein, the step of methylating dimethyl (S)-2-tert-butyloxycarbonylamino-glutarate i into dimethyl (2S,4S)-2-tert-butyloxycarbonylamino-4-methyl-glutarate ii includes under main operating conditions of a low temperature less than -78°C, adding dimethyl (S)-2-tert-butyloxycarbonylamino-glutarate i to the LiHMDS solution, and then adding iodomethane thereto for the methylation reaction, after the completion of the reaction, adding methanol and an acid water (such as hydrochloric acid) successively for quenching, adding an extraction solvent (such as ethyl acetate, and methyl tert-butyl ether) for extraction and layering, concentrating the organic phase under reduced pressure to dryness, then adding a crystallization solvent (such as methyl tert-butyl ether, ethyl acetate, and n-heptane) for slurrying and crystallizing, filtering and drying same to obtain dimethyl (2S,4S)-2-tert-butyloxycarbonylamino-4-methyl-glutarate ii.

The synthesis methods for methylating dimethyl (S)-2-tert-butyloxycarbonylamino-glutarate publicly reported in the prior art are as follows:
Route 1 is a method described in the patent of a piperidine derivative compound [Patent No.: CN102093260B], [Application No.: US 20100152452A1]. The specific route is as follows:

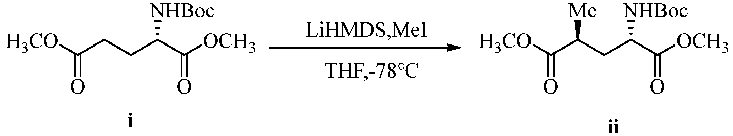

### Synthetic route 1

In this route, compound i is used as the starting raw material, the deprotonation of the carbonyl alpha carbon of compound i is first carried out under the action of LiHMDS, i.e. using the LiHMDS reagent to pull out the H atom on the carbonyl alpha carbon of compound i, the obtained intermediate is subjected to methylation with iodomethane, then a solution of compound ii is obtained after quenching. The route uses a LiHMDS reagent, which requires low temperature (less than or equal to -78 °C), the reaction conditions are harsh and difficult to operate, this phenomenon is particularly obvious in the scale-up production. Moreover, the processing operation is complex, and needs to be quenched with an alcohol and an acid water solution successively at low temperature, an alcohol solvent is introduced in the quenching process, which is not conducive to the separation and purification of the reaction solvent, and the reaction time is long, a laboratory small test reaction of a conventional 30-60 g scale usually takes 2 days, and a workshop production of 80-100 kg scale takes 3-4 days.

Route 2 is a method described in the preparation patent [Application No.: US005731348A]. The specific synthesis method is as follows:

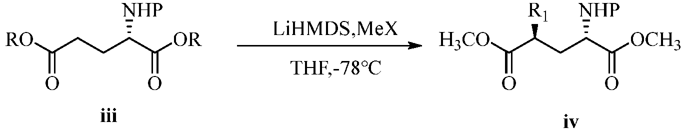

### Synthetic route 2

The reaction mechanism of synthetic route 2 is consistent with that of synthetic route 1. In this route, the hydrogen atom in compound iii is first pulled out with an organic lithium reagent (LiHMDS) at an ultra-low temperature of -78 °C, and then the resulting product is connected to an alkylation skeleton through a haloalkane still at a reaction temperature of -78 °C for a reaction time of 24 h, then the compound iv is obtained by quenching and purifying with alcohol and acid water successively. This route still cannot solve the problem as described in route 1.

### SUMMARY

In view of the shortcomings existing in the above preparation methods, the present disclosure provides a method for continuously synthesizing an intermediate dimethyl (2S,4S)-2-tert-butyloxycarbonylamino-4-methyl-glutarate ii by a microreactor. The method has advantages of mild reaction conditions, shorter reaction time, good safety, simple operation, economy and environmental protection, high yield, good product quality and ease of industrial production.

According to a first aspect of the present disclosure 1, a method for continuously synthesizing compound of formula ii using a microreactor is provided, wherein it comprises the following steps:
S1) reacting compound i with a hydrogen-pulling reagent and a methylation reagent at -78 °C to - 20 °C, in an inert solvent to obtain a reaction solution containing compound of formula ii;
S2) quenching the reaction solution obtained in step S1) with an acid solution at -20 °C to 40 °C to obtain the compound of formula ii;

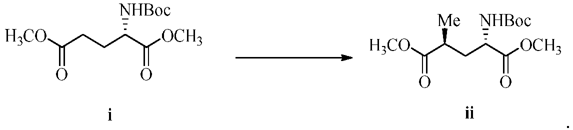

In another preferred embodiment, in step S1), the hydrogen pulling reagent is a lithium reagent, preferably the hydrogen pulling reagent is selected from lithium hexamethyldisilazide (LiHDMS), lithium diisopropylamide (LDA), butyl lithium, sodium hexamethyldisilazide (NaHMDS), preferably lithium hexamethyldisilazide (LiHDMS).

In another preferred embodiment, in step S1), the methylation reagent is selected from iodomethane, dimethyl sulfate, dimethyl carbonate, bromomethane, and preferably iodomethane.

In another preferred embodiment, the microreactor comprises a reaction module and a post-processing module, wherein the step S1) is carried out in the reaction module and the step S2) is carried out in the post-processing module.

In another preferred embodiment, the reaction module comprises a reaction unit A and/or a reaction unit B, and the step S1) comprises the following steps:
i-1) pre-cooling a solution of compound i and a solution of hydrogen-pulling reagent and then feeding same into the unit A for reacting at -78 °C to -20 °C;
i-2) flowing the reaction solution of step i-1) into the unit B and then feeding a solution of methylation reagent into the unit B for reacting at -78 °C to -20 °C to obtain a reaction solution containing compound ii;
or i'- 1) premixing the solution of compound i and the solution of methylation reagent and then feeding same with the solution of hydrogen-pulling reagent into the unit A or B for reacting at -78 °C to -20 °C to obtain the reaction solution of compound ii;
or i"- 1) premixing the solution of compound i and the solution of methylation reagent in the unit A and then feeding same with the solution of hydrogen-pulling reagent into the unit B for reacting at -78 °C to -20 °C to obtain the reaction solution of compound ii.

In another preferred embodiment, the solvent of the solution of compound i is selected from tetrahydrofuran, methyltetrahydrofuran, ethyl acetate, or a combination thereof.

In another preferred embodiment, the solvent of the solution of hydrogen-pulling reagent is selected from tetrahydrofuran, methyltetrahydrofuran, ethyl acetate, or a combination thereof.

In another preferred embodiment, the solvent of the solution of methylation reagent is selected from tetrahydrofuran, methyltetrahydrofuran, ethyl acetate, or a combination thereof.

In another preferred embodiment, the concentration of the solution of compound i is 200-400 g/L, preferably 280-350 g/L, and more preferably 300-320 g/L.

In another preferred embodiment, the solution of hydrogen-pulling reagent is a solution of lithium reagent, preferably a solution of lithium hexamethyldisilazide (a solution of LiHMDS in tetrahydrofuran), preferably the concentration of the solution of hydrogen-pulling reagent is 0.8-1.3 mol/L, preferably 0.8-1.0 mol/L.

In another preferred embodiment, the molar ratio of compound i to lithium reagent (such as lithium hexamethyldisilazide (LiHMDS)) is 1:1.7-3.0, preferably 1:2.0.

In another preferred embodiment, the concentration of the solution of methylation reagent is 500-700 g/L, preferably 550-650 g/L.

In another preferred embodiment, in step i-1), the residence time of the mixed materials in the unit A is not more than 200 seconds, preferably 5 to 190 seconds, more preferably 10 to 150 seconds, and more preferably 10 to 80 seconds.

In another preferred embodiment, in step i'-1), the residence time of the mixed materials in the unit A/unit B is not more than 400 seconds, preferably 5 to 350 seconds, and more preferably 100 to 300 seconds.

In another preferred embodiment, the molar ratio of compound i to methylation reagent is 1:1.0-3.0, preferably 1:1.2.

In another preferred embodiment, in step i-2), the residence time of the mixed materials in the unit B is not more than 300 seconds, preferably 30 to 200 seconds, more preferably 65 to 150 seconds, and more preferably 75 to 120 seconds.

In another preferred embodiment, in step i-1), the reaction temperature of the unit A is -50 to - 20 °C, preferably -40 to -20 °C.

In another preferred embodiment, in step i'-1), the reaction temperature of the unit A is preferably -60 to -20 °C, and more preferably -60 to -40 °C.

In another preferred embodiment, in step i-2), the reaction temperature of the unit B is -50 to - 20 °C, preferably -30 to -20 °C.

In another preferred embodiment, the post-processing module includes a reaction unit C, and the step S2) includes the following steps:
i-3) flowing the obtained reaction solution of compound ii into the unit C, and at the same time feeding an acid solution into the unit C for quenching reaction at -78 °C to -20 °C to obtain a stable quenching reaction solution containing compound ii.

In another preferred embodiment, the solvent of the acid solution is selected from water, alcohol, or a combination thereof, and the acid is selected from hydrochloric acid, sulfuric acid, or a combination thereof.

In another preferred embodiment, the alcohol is selected from methanol, ethanol, or a combination thereof.

In another preferred embodiment, the acid solution is selected from aqueous hydrochloric acid solution or aqueous sulfuric acid solution.

In another preferred embodiment, the acid solution comprises a salt or no salt, preferably the salt is NaCl, KCI, NH₄Cl, or a combination thereof.

In another preferred embodiment, the acid solution is hydrochloric acid in aqueous sodium chloride solution.

In another preferred embodiment, the step S2) also includes steps of static stratification, crystallization and filtration.

In another preferred embodiment, the purity of the compound of formula ii obtained in step S2) after post-processing is greater than 90%, preferably greater than 94%, more preferably greater than 95%, more preferably greater than 96%, more preferably greater than 97%, and more preferably greater than 98%.

In another preferred embodiment, the molar ratio of compound i to acid is 1:5.0-10.0.

In another preferred embodiment, the residence time of the reaction solution in the unit C is not more than 100 seconds, preferably 1 to 50 seconds, and more preferably 1 to 15 seconds.

In another preferred embodiment, the microreactor is composed of one or more of the following reactors: Corning reactor, micro-well (silicon carbide, Hastelloy, 316L) reactor, and Shen's (Hastelloy, 316L) reactor.

It should be understood that within the scope of the present disclosure, the above technical features of the present disclosure and the technical features described in detail below (such as in the embodiments) can be combined with each other to form a new or preferred technical solution. It will not be repeated herein in order to avoid redundancy.

### BRIEF DESCRIPTION OF THE DRAWINGS

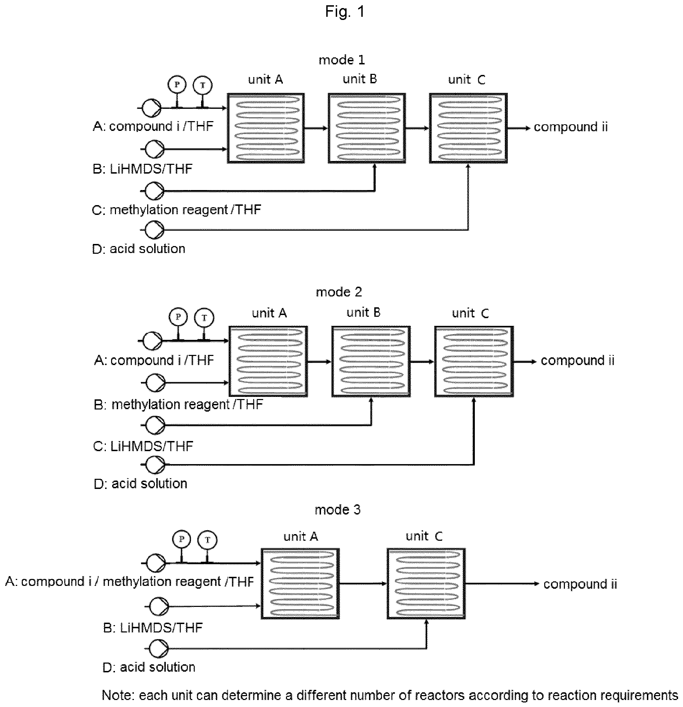

Fig. 1 shows the flow diagram of the method according to the present disclosure.

### DETAILED DESCRIPTION

Through extensive and intensive researches, the inventor of the present application unexpectedly developed a method for continuously synthesizing dimethyl (2S,4S)-2-tert-butyloxycarbonylamino-4-methyl-glutarate using a microchannel reactor. The method improves the selectivity and conversion rate of the reaction, and the conversion rate of dimethyl (2S,4S)-2-tert-butyloxycarbonylamino-4-methyl-glutarate is increased to more than 95% and the yield is increased to more than 85%; avoids the use of a solvent such as methanol, and methyl tert-butyl ether, etc., in the intermittent reaction process, which simplifies the post-processing method, shortens the overall operation time from about 24 hours to a few minutes, greatly improving the production efficiency, and realizing the continuity and automation of the whole process; and thus makes the product have high purity and high yield, which is suitable for industrial production.

In the present disclosure, the "compound of formula ii", "dimethyl (2S,4S)-2-tert-butyloxycarbonylamino-4-methyl-glutarate" and "quinolones compound intermediate" have the same meaning and can be used interchangeably.

In the present disclosure, the "main raw material" and "compound i" have the same meaning.

### Microreactor

The microreactor includes a microchannel reactor, a micro heat transfer reactor, and a micro mixing reactor, etc., and it is a minitype reactor with a feature size of 10 to 1,000 µm manufactured by precision machining technology. The "micro" of the microreactor neither specially refer to the overall dimension size of the microreactor equipment, nor the small output of product by the microreactor equipment, but means that the channel of the process fluid is at the micron level, and the microreactor can comprise hundreds of thousands of microchannels. In this application, the annual flux of compound ii can reach 5 tons/year by using laboratory microreactor (10 ml), and 150 to 250 tons/year by using industrial microreactor (300 ml). The microreactor used in this application can be one or more of Corning reactor, micro-well (silicon carbide, Hastelloy, 316L) reactor, and Shen's (Hastelloy, 316L) reactor.

### Method for continuously synthesizing compound of formula ii using a microreactor

The present disclosure uses a microreactor to continuously synthesize the compound of formula ii, preferably dimethyl (2S,4S)-2-tert-butyloxycarbonylamino-4-methyl-glutarate ii can be obtained by using dimethyl (S)-2-tert-butyloxycarbonylamino-glutarate i as the starting material, followed by the steps of hydrogen pulling reaction, methylation reaction and quenching reaction,

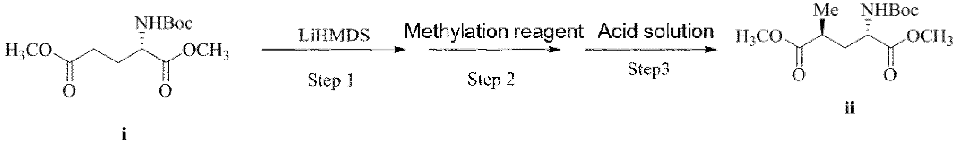

Preferably, in the present disclosure, the compound ii is prepared by the microchannel one-pot method in a continuous flow. The microreactor is divided into three units A, B and C, and the specific reaction route is as follows: 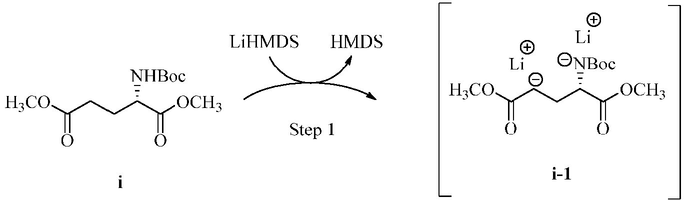 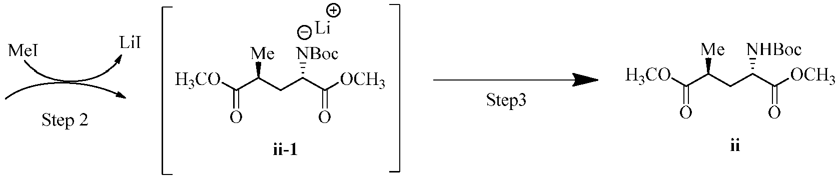 1) mixing the solution of compound i with an organic lithium reagent in the unit A at a reaction temperature of -50 to -20 °C; 2) feeding the intermediate i-1 and a methylation reagent into the unit B at the same time for reacting to obtain a reaction solution of intermediate ii-1; 3) feeding the reaction solution containing intermediate ii-1 and an acid solution (such as an aqueous hydrochloric acid solution) into the unit C for reacting to obtain the intermediate compound ii with a reaction temperature of unit C being -20 to 20 °C, wherein the flow diagram of which is as shown in mode 1 of Fig. 1.

Preferably, the present application can also be carried out by the method as shown in the mode 2 or 3 of the flow diagram of Fig. 1:
i'- 1) premixing the solution of compound i and the solution of methylation reagent and then feeding same with the solution of hydrogen-pulling reagent into the unit A or B for reacting at -78 °C to -20 °C to obtain the reaction solution of compound ii;
or i"- 1) premixing the solution of compound i and the solution of methylation reagent in the unit A and then feeding same with the solution of hydrogen-pulling reagent into the unit B for reacting at -78 °C to -20 °C to obtain the reaction solution of compound ii.

Preferably, the organic solvent for dissolving compound i is tetrahydrofuran and ethyl acetate.

Preferably, the reagent for quenching compound ii adopts an aqueous hydrochloric acid solution and an aqueous sulfuric acid solution.

Preferably, the molar ratio of compound i to lithium reagent (such as lithium hexamethyldisilazide (LiHMDS)) is 1: 1.7-3.0, preferably 1: 2.0, the molar ratio of methylation reagent to compound i is 1: 1.0-3.0, preferably 1: 1.2, and the molar ratio of hydrochloric acid/sulfuric acid solution to compound i is 1: 5.0-10.0, 1: 2.5-5.0, and preferably 1: 5.5 and 1: 3.0.

For the above three units A, B and C, a delayed tubular reactor can be added behind the microreactor according to the need of reaction residence time.

Preferably, the specific steps of the present disclosure are as follows:
1') dissolving compound i with tetrahydrofuran or ethyl acetate until the feed solution is clear and ready for use, precooling the solution of compound i in tetrahydrofuran or ethyl acetate and the solution of hexamethyldisilazane, then pumping same into the unit A of the microreactor, and reacting at - 50 to - 20 °C;
wherein, the concentration of the solution of compound i in tetrahydrofuran/ethyl acetate is 200-400 g/L, preferably 280-350 g/L; and the concentration of the solution of lithium hexamethyldisilazide (the solution of LiHMDS in tetrahydrofuran) is 0.8-1.3 mol/L, preferably 0.8-1.0 mol/L;
the molar ratio of compound i to lithium hexamethyldisilazide (LiHMDS) is 1:1.7-3.0, preferably 1:2.0;
the residence time of the mixed materials in the unit A is not more than 200 seconds, preferably 10-80 seconds;
2') flowing the reaction solution obtained in step 1') into the unit B of the microreactor, feeding the solution of methylation reagent in tetrahydrofuran/ethyl acetate into the unit B, and fully reacting at a reaction temperature of -50 to -20 °C (preferably -30 to - 20 °C) to obtain a reaction solution containing compound ii;
wherein the methylation reagent is one of iodomethane, dimethyl sulfate and dimethyl carbonate; preferably iodomethane.

The concentration of the solution of methylation reagent in tetrahydrofuran is 500-700 g/L, preferably 550-650 g/L;
The molar ratio of compound i to methylation reagent is 1:1.0-3.0, preferably 1:1.2, the residence time in the unit B is not more than 300 seconds, preferably 65-150 seconds;
3') directly flowing the reaction solution obtained in step 2') into the unit C of the microreactor without treatment, and pumping an acid solution (such as an aqueous hydrochloric acid solution) into the unit C, reacting until completion at a reaction temperature of -20 to 20 °C (preferably 10 to 20 °C), after the reaction is completed, flowing the reaction mixture out of the microreactor and into the reaction tank to obtain the reaction liquid of compound ii.

Wherein, the concentration of the aqueous acid solution is 100-400 g/L, preferably 160-260 g/L, and the molar ratio of compound i to acid is 1:5.0-10.0, preferably 1:5.5;
the residence time of the reaction solution in the unit C is not more than 100 seconds, preferably 1 to 50 seconds, and more preferably 1 to 15 seconds.

The microreactor used in the present disclosure includes Corning reactor, micro-well (silicon carbide, Hastelloy, 316L) reactor, and Shen's (Hastelloy, 316L) reactor. According to the common sense of use, the size of the reactor can be appropriately increased for industrial scale-up production.

### Use of compound ii

In the present disclosure, the obtained dimethyl (2S,4S)-2-tert-butyloxycarbonylamino-4-methyl-glutarate ii further undergoes reaction steps such as ammonolysis, cyanidation, cyclization, etc., to obtain a piperidine derivative iv, and further, which can undergo condensation, hydrolysis and salifying reactions with 1-cyclopropyl-7-fluoro-8-methoxy-4-oxo-1,4-dihydro-quinolin-3-carboxylic acid diacetate boric anhydride iii to produce the finished product of Nemonoxacin malate.

### Compared with the prior art, the preset disclosure has the following main advantages:

(1) The present disclosure combines the advantages of high efficient mass transfer and heat transfer of the microchannel reactor to improve the conversion rate and selectivity of the reaction, and the conversion rate of compound ii is increased to more than 95%, and the yield of product can be increased to more than 85%, greatly reducing the difficulty in subsequent purification and separation.
(2) The present disclosure avoids the use of solvents such as methanol, and methyl tert-butyl ether, etc., in the reaction and post-processing process, such that the organic phase can be subjected to the stratification operation directly layer in the post-processing process, solving a number of problems such as cumbersome operations and difficult recovery of waste liquid, etc., in the post-processing process of an intermittent process.
(3) The reaction time of the present disclosure is shortened from 24 hours to minute level, and the residue of raw materials is reduced from the original 7-15% to 3-5%, which greatly reduces the energy consumption of the reaction and the economic cost of the process.
(4) The present disclosure greatly improves the reaction temperature, avoids the reaction under ultra-low temperature, and reduces energy consumption and safety risks.

The present disclosure will be further described below in combination with specific examples. It should be understood that these examples are only used to explain the present disclosure rather than limiting the scope of the present disclosure. The experimental methods without indicating specific conditions in the following examples are generally in accordance with the conventional conditions or the conditions recommended by the manufacturers. Unless otherwise stated, percentages and parts are calculated by weight.

### Example 1

190 g of main raw materials, 162 g of bromomethane and 635 g of tetrahydrofuran were added into a 1 L three-necked flask to prepare a solution of compound i/bromomethane in tetrahydrofuran, which was flowed into the unit A module at a flow rate of 32 ml/min, and lithium hexamethyldisilazide with a concentration of 1.2 mol/L was let to flow into the unit A at a flow rate of 50 ml/min at the same time, the reaction residence time was 267 seconds, and the reaction temperature of the reaction unit was controlled at -55 °C. After the reaction solution flowed out of the unit A, the prepared 200 g/L of hydrochloric acid in aqueous sodium chloride solution was simultaneously pumped into the unit C at a flow rate of 52 ml/min, the residence time of the reaction unit was 5.8 seconds, and the reaction temperature of the reaction unit was controlled at 0 °C. The liquid material flowing out of the unit was stood still for layering, the organic layer was rotary evaporated to nearly dryness, n-heptane was added therein, then cooled and crystallized, suction filtered to obtain the intermediate compound ii. The purity of this batch of samples was 97.42%, with the epimer of 1.22%, and a total yield of 83.74%.

### Example 2

A solution of 380 g/L of main raw material in tetrahydrofuran was prepared in a 1 L three-necked flask, and flowed into the unit A at a flow rate of 21 ml/min, additionally a prepared solution of 620 g/L of iodomethane in tetrahydrofuran was flowed into the unit A at a flow rate of 13.0 ml/min at the same time. The residence time of the reaction unit was 8 seconds, and the reaction temperature of the reaction unit was controlled at -78 °C. After the reaction solution flowed out of the unit A, lithium hexamethyldisilazide with a concentration of 1.2 mol/L was flowed into the unit B at a flow rate of 50 ml/min. The reaction residence time was 274 seconds, and the reaction temperature was controlled at -50 °C. After the reaction solution flowed out of the unit B, the prepared 200 g/L of hydrochloric acid in aqueous sodium chloride solution was simultaneously pumped into the unit C at a flow rate of 35 ml/min, the residence time of the reaction unit was 5.1 seconds, and the reaction temperature of the reaction unit was controlled at 40 °C. The liquid material flowing out of the unit was stood still for layering, the organic layer was rotary evaporated to nearly dryness, n-heptane was added therein, then cooled and crystallized, suction filtered to obtain the intermediate compound ii. The purity of this batch of samples was 95.17%, with the epimer of 1.99%, and a total yield of 81.11%.

### Example 3

A solution of 320 g/L of main raw material in tetrahydrofuran was prepared in a 1 L three-necked flask, and flowed into the unit A at a flow rate of 21 ml/min, additionally lithium hexamethyldisilazide with a concentration of 1.2 mol/L was flowed into the unit A at a flow rate of 50 ml/min at the same time. The reaction residence time was 194 seconds, and the reaction temperature was controlled at -50 °C. After the reaction solution flowed out of the unit A, a prepared solution of 320 g/L of iodomethane in tetrahydrofuran was flowed into the unit B at a flow rate of 13.0 ml/min at the same time. The residence time of the reaction unit was 297 seconds, and the reaction temperature of the reaction unit was controlled at -20 °C. After the reaction solution flowed out of the unit B, the prepared 200 g/L of hydrochloric acid in aqueous sodium chloride solution was simultaneously pumped into the unit C at a flow rate of 35 ml/min, the residence time of the reaction unit was 3.9 seconds, and the reaction temperature of the reaction unit was controlled at 0 °C. The liquid material flowing out of the unit was stood still for layering, the organic layer was rotary evaporated to nearly dryness, n-heptane was added therein, then cooled and crystallized, suction filtered to obtain the intermediate compound ii. The purity of this batch of samples was 96.41%, with the epimer of 1.17%, and a total yield of 80.6%.

### Example 4

A solution of 320 g/L of main raw material in tetrahydrofuran was prepared in a 1 L three-necked flask, and flowed into the unit A at a flow rate of 21 ml/min, additionally lithium hexamethyldisilazide with a concentration of 1.2 mol/L was flowed into the unit A at a flow rate of 50 ml/min at the same time. The reaction residence time was 152 seconds, and the reaction temperature was controlled at -40 °C. After the reaction solution flowed out of the unit A, a prepared solution of 725 g/L of bromomethane in tetrahydrofuran was flowed into the unit B at a flow rate of 16 ml/min at the same time. The residence time of the reaction unit was 164.5 seconds, and the reaction temperature of the reaction unit was controlled at -50 °C. After the reaction solution flowed out of the unit B, the prepared 400 g/L of hydrochloric acid in aqueous ammonium chloride solution was simultaneously pumped into the unit C at a flow rate of 18 ml/min, the residence time of the reaction unit was 98 seconds, and the reaction temperature of the reaction unit was controlled at -20 °C. The liquid material flowing out of the unit was stood still for layering, the organic layer was rotary evaporated to nearly dryness, n-heptane was added therein, then cooled and crystallized, suction filtered to obtain the intermediate compound ii. The purity of this batch of samples was 95.94%, with the epimer of 1.44%, and a total yield of 84.85%.

### Example 5

A solution of 380g/L of main raw material in tetrahydrofuran was prepared in a 1 L three-necked flask, and flowed into the unit A at a flow rate of 21 ml/min, additionally lithium hexamethyldisilazide with a concentration of 1.2 mol/L was flowed into the unit A at a flow rate of 50 ml/min at the same time. The reaction residence time was 102 seconds, and the reaction temperature was controlled at -35 °C. After the reaction solution flowed out of the unit A, a prepared solution of 810 g/L of bromomethane in tetrahydrofuran was flowed into the unit B at a flow rate of 11 ml/min at the same time. The residence time of the reaction unit was 80.5 seconds, and the reaction temperature of the reaction unit was controlled at -35 °C. After the reaction solution flowed out of the unit B, the prepared 100 g/L of aqueous hydrochloric acid solution was simultaneously pumped into the unit C at a flow rate of 65 ml/min, the residence time of the reaction unit was 2.7 seconds, and the reaction temperature of the reaction unit was controlled at 20 °C. The liquid material flowing out of the unit was stood still for layering, the organic layer was rotary evaporated to nearly dryness, n-heptane was added therein, then cooled and crystallized, suction filtered to obtain the intermediate compound ii. The purity of this batch of samples was 93.45%, with the epimer of 1.79%, and a total yield of 83.2%.

### Example 6

A solution of 200 g/L of main raw material in tetrahydrofuran was prepared in a 1 L three-necked flask, and flowed into the unit A at a flow rate of 30 ml/min, additionally lithium hexamethyldisilazide with a concentration of 0.9 mol/L was flowed into the unit A at a flow rate of 75 ml/min at the same time. The reaction residence time was 64 seconds, and the reaction temperature was controlled at - 30 °C. After the reaction solution flowed out of the unit A, a prepared solution of 210 g/L of iodomethane in tetrahydrofuran was flowed into the unit B at a flow rate of 15 ml/min at the same time. The residence time of the reaction unit was 79 seconds, and the reaction temperature of the reaction unit was controlled at -35 °C. After the reaction solution flowed out of the unit B, the prepared 200 g/L of aqueous hydrochloric acid solution was simultaneously pumped into the unit C at a flow rate of 35 ml/min, the residence time of the reaction unit was 4.9 seconds, and the reaction temperature of the reaction unit was controlled at 0 °C. The liquid material flowing out of the unit was stood still for layering, the organic layer was rotary evaporated to nearly dryness, n-heptane was added therein, then cooled and crystallized, suction filtered to obtain the intermediate compound ii. The purity of this batch of samples was 98.45%, with the epimer of 0.44%, and a total yield of 81.7%.

### Example 7

A solution of 400g/L of main raw material in tetrahydrofuran was prepared in a 1 L three-necked flask, and flowed into the unit A at a flow rate of 20ml/min, additionally lithium hexamethyldisilazide with a concentration of 0.8mol/L was flowed into the unit A at a flow rate of 80ml/min at the same time. The reaction residence time was 10.1 seconds, and the reaction temperature was controlled at -45 °C. After the reaction solution flowed out of the unit A, a prepared solution of 350 g/L of iodomethane in tetrahydrofuran was flowed into the unit B at a flow rate of 13 ml/min at the same time. The residence time of the reaction unit was 76 seconds, and the reaction temperature of the reaction unit was controlled at -45 °C. After the reaction solution flowed out of the unit B, the prepared 200 g/L of aqueous hydrochloric acid solution was simultaneously pumped into the unit C at a flow rate of 35 ml/min, the residence time of the reaction unit was 34.7 seconds, and the reaction temperature of the reaction unit was controlled at 10 °C. The liquid material flowing out of the unit was stood still for layering, the organic layer was rotary evaporated to nearly dryness, n-heptane was added therein, then cooled and crystallized, suction filtered to obtain the intermediate compound ii. The purity of this batch of samples was 96.17%, with the epimer of 1.24%, and a total yield of 87.14%.

### Example 8

A solution of 380 g/L of main raw material in tetrahydrofuran was prepared in a 1 L three-necked flask, and flowed into the unit A at a flow rate of 11ml/min, additionally lithium hexamethyldisilazide with a concentration of 0.9 mol/L was flowed into the unit A at a flow rate of 35ml/min at the same time. The reaction residence time was 16.0 seconds, and the reaction temperature was controlled at -25 °C. After the reaction solution flowed out of the unit A, a prepared solution of 650 g/L of iodomethane in tetrahydrofuran was flowed into the unit B at a flow rate of 7 ml/min at the same time. The residence time of the reaction unit was 160.1 seconds, and the reaction temperature of the reaction unit was controlled at -25 °C. After the reaction solution flowed out of the unit B, the prepared 200 g/L of aqueous hydrochloric acid solution was simultaneously pumped into the unit C at a flow rate of 17 ml/min, the residence time of the reaction unit was 14.9 seconds, and the reaction temperature of the reaction unit was controlled at 0 °C. The liquid material flowing out of the unit was stood still for layering, the organic layer was rotary evaporated to nearly dryness, n-heptane was added therein, then cooled and crystallized, suction filtered to obtain the intermediate compound ii. The purity of this batch of samples was 95.21%, with the epimer of 1.33%, and a total yield of 88.4%.

### Example 9

A solution of 380 g/L of main raw material in tetrahydrofuran was prepared in a 1 L three-necked flask, and flowed into the unit A at a flow rate of 30ml/min, additionally lithium hexamethyldisilazide with a concentration of 1.0mol/L was flowed into the unit A at a flow rate of 60ml/min at the same time. The reaction residence time was 4.5 seconds, and the reaction temperature was controlled at -25 °C. After the reaction solution flowed out of the unit A, a prepared solution of 650 g/L of iodomethane in tetrahydrofuran was flowed into the unit B at a flow rate of 12 ml/min at the same time. The residence time of the reaction unit was 50.7 seconds, and the reaction temperature of the reaction unit was controlled at -25 °C. After the reaction solution flowed out of the unit B, the prepared 200 g/L of aqueous hydrochloric acid solution was simultaneously pumped into the unit C at a flow rate of 51 ml/min, the residence time of the reaction unit was 3.1 seconds, and the reaction temperature of the reaction unit was controlled at -20 °C. The liquid material flowing out of the unit was stood still for layering, the organic layer was rotary evaporated to nearly dryness, n-heptane was added therein, then cooled and crystallized, suction filtered to obtain the intermediate compound ii. The purity of this batch of samples was 94.33%, with the epimer of 1.71%, and a total yield of 87.4%.

### Example 10

A solution of 300g/L of main raw material in tetrahydrofuran was prepared in a 1 L three-necked flask, and flowed into the unit A at a flow rate of 21ml/min, additionally lithium hexamethyldisilazide with a concentration of 1.3mol/L was flowed into the unit A at a flow rate of 35ml/min at the same time. The reaction residence time was 75 seconds, and the reaction temperature was controlled at - 25 °C. After the reaction solution flowed out of the unit A, a prepared solution of 650 g/L of iodomethane in tetrahydrofuran was flowed into the unit B at a flow rate of 10 ml/min at the same time. The residence time of the reaction unit was 76 seconds, and the reaction temperature of the reaction unit was controlled at -25 °C. After the reaction solution flowed out of the unit B, the prepared 100 g/L of aqueous sulfuric acid solution was simultaneously pumped into the unit C at a flow rate of 35 ml/min, the residence time of the reaction unit was 3.2 seconds, and the reaction temperature of the reaction unit was controlled at 0 °C. The liquid material flowing out of the unit was stood still for layering, the organic layer was rotary evaporated to nearly dryness, n-heptane was added therein, then cooled and crystallized, suction filtered to obtain the intermediate compound ii. The purity of this batch of samples was 90.41%, with the epimer of 4.33%, and a total yield of 78.2%.

### Example 11

A solution of 380 g/L of main raw material in ethyl acetate was prepared in a 1 L three-necked flask, and flowed into the unit A at a flow rate of 21 ml/min, additionally lithium hexamethyldisilazide with a concentration of 1.0 mol/L was flowed into the unit A at a flow rate of 49 ml/min at the same time. The reaction residence time was 34 seconds, and the reaction temperature was controlled at - 20 °C. After the reaction solution flowed out of the unit A, a prepared solution of 650g/L of iodomethane in tetrahydrofuran was flowed into the unit B at a flow rate of 13 ml/min at the same time. The residence time of the reaction unit was 116.9 seconds, and the reaction temperature of the reaction unit was controlled at -25 °C. After the reaction solution flowed out of the unit B, the prepared 200 g/L of aqueous hydrochloric acid solution was simultaneously pumped into the unit C at a flow rate of 35 ml/min, the residence time of the reaction unit was 68 seconds, and the reaction temperature of the reaction unit was controlled at -10 °C. The liquid material flowing out of the unit was stood still for layering, the organic layer was rotary evaporated to nearly dryness, n-heptane was added therein, then cooled and crystallized, suction filtered to obtain the intermediate compound ii. The purity of this batch of samples was 90.33%, with the epimer of 3.12%, and a total yield of 78.1%.

### Example 12

A solution of 380 g/L of main raw material in tetrahydrofuran was prepared in a 1 L three-necked flask, and flowed into the unit A at a flow rate of 21ml/min, additionally lithium hexamethyldisilazide with a concentration of 0.8mol/L was flowed into the unit A at a flow rate of 65ml/min at the same time. The reaction residence time was 10.0 seconds, and the reaction temperature was controlled at -35 °C. After the reaction solution flowed out of the unit A, a prepared solution of 578 g/L of dimethyl sulfate in tetrahydrofuran was flowed into the unit B at a flow rate of 13 ml/min at the same time. The residence time of the reaction unit was 121.4 seconds, and the reaction temperature of the reaction unit was controlled at -45 °C. After the reaction solution flowed out of the unit B, the prepared 200 g/L of aqueous hydrochloric acid solution was simultaneously pumped into the unit C at a flow rate of 48 ml/min, the residence time of the reaction unit was 3.8 seconds, and the reaction temperature of the reaction unit was controlled at -10 °C. The liquid material flowing out of the unit was stood still for layering, the organic layer was rotary evaporated to nearly dryness, n-heptane was added therein, then cooled and crystallized, suction filtered to obtain the intermediate compound ii. The purity of this batch of samples was 95.33%, with the epimer of 1.49%, and a total yield of 87.1%.

### Comparative example 1

A solution of 1M LiHMDS in tetrahydrofuran (1500 ml) was added to a 5 L four-necked flask at -78 °C under the protection of nitrogen, then a solution of compound i (a solution of 210 g compound i in 1000 ml of dry tetrahydrofuran) was added dropwise thereto at no less than -60 °C, and then stirred at -78 °C for 6.5 h. lodomethane (a solution of 176 g iodomethane in 100 ml anhydrous tetrahydrofuran) was added to the formed solution at -60 °C. The reaction was stirred at -78 °C for 7.0 h, then the reaction was quenched with methanol (35 g) at a condition of -60 °C and 2N hydrochloric acid (1500 ml) at a condition of -25 °C. Toluene (1500 ml) was added into the formed solution, the mixture was stirred for 0.5 to 1.0 h, the organic layer was separated, and treated with a solution of sodium thiosulfate (a solution of 175 g sodium thiosulfate in 1000 ml water) for 30 minutes with stirring, during this time, the color of which changed from dark brown to light yellow. The organic layer was evaporated under vacuum, n-heptane was added, then cooled and crystallized, suction filtered to obtain the intermediate compound ii. The purity of this batch of samples was 94.74%, with the epimer of 1.94%, and a total yield of 84.2%.

### Comparative example 2

A solution of 1M LiHMDS in tetrahydrofuran (500 ml) was added to a 2 L four-necked flask at - 78 °C under the protection of nitrogen, then a solution of compound i (a solution of 70 g compound i in 350 ml of dry tetrahydrofuran) was added dropwise thereto at no less than -60 °C, and then stirred at -78 °C for 8.5 h. lodomethane (a solution of 60 g iodomethane in 35 ml anhydrous tetrahydrofuran) was added to the formed solution at -65 °C. The reaction was stirred at -78 °C for 12.0 h, then the reaction was quenched with methanol (35 g) at a condition of -78 °C and 2N hydrochloric acid (1500 ml) at a condition of -25 °C. Methyl tert-butyl ether (350 ml) was added into the formed solution, the mixture was stirred for 0.5 to 1.0 h, the organic layer was separated, and treated with a solution of sodium thiosulfate (a solution of 60 g sodium thiosulfate in 350 ml water) for 30 minutes with stirring, during this time, the color of which changed from dark brown to light yellow. The organic layer was evaporated under vacuum, n-heptane was added, then cooled and crystallized, suction filtered to obtain the intermediate compound ii. The purity of this batch of samples was 95.19%, with the epimer of 1.94%, and a total yield of 84.7%.

### Comparative example 3

A solution of 1M LiHMDS in tetrahydrofuran (500 ml) was added to a 2 L four-necked flask at - 78 °C under the protection of nitrogen, then a solution of compound i (a solution of 70 g compound i in 350 ml of dry tetrahydrofuran) was added dropwise thereto at no less than -60 °C, and then stirred at -78 °C for 8.5 h. lodomethane (a solution of 60 g iodomethane in 35 ml anhydrous tetrahydrofuran) was added to the formed solution at -65 °C. The reaction was stirred at -78 °C for 12.0 h, and then the reaction was quenched directly with 2N hydrochloric acid (1500 ml) at -78 °C (the system was exothermic significantly, the quenching process was severely iced, and the properties and states of the reaction liquid were poor). Methyl tert-butyl ether (350 ml) was added into the formed solution, the mixture was stirred for 0.5 to 1.0 h, the organic layer was separated, and treated with a solution of sodium thiosulfate (a solution of 60 g sodium thiosulfate in 350 ml water) for 30 minutes with stirring, during this time, the color of which changed from dark brown to light yellow. The organic layer was evaporated under vacuum, n-heptane was added, then cooled and crystallized, suction filtered to obtain the intermediate compound ii. The purity of this batch of samples was 80.71%, with the epimer of 6.71%, and a total yield of 70.5%.

## Claims

1. A method for continuously synthesizing compound of formula ii using a microreactor, wherein it comprises the following steps:
S1) reacting compound i with a hydrogen-pulling reagent and a methylation reagent at -78 °C to - 20 °C, in an inert solvent to obtain a reaction solution containing compound of formula ii;
S2) quenching the reaction solution obtained in step S1) with an acid solution at -20 °C to 40 °C to obtain the compound of formula ii;

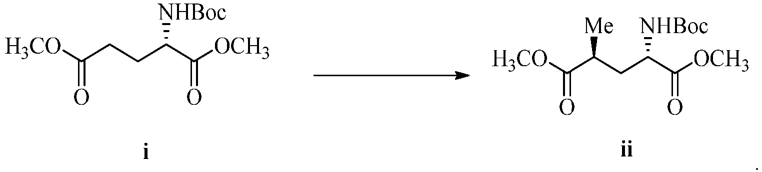

2. The method according to claim 1, wherein the microreactor comprises a reaction module and a post-processing module, wherein the step S1) is carried out in the reaction module and the step S2) is carried out in the post-processing module.

3. The method according to claim 2, wherein the reaction module comprises a reaction unit A and/or a reaction unit B, and the step S1) comprises the following steps:
i-1) pre-cooling a solution of compound i and a solution of hydrogen-pulling reagent and then feeding same into the unit A for reacting at -78 °C to -20 °C;
i-2) flowing the reaction solution of step i-1) into the unit B and then feeding a solution of methylation reagent into the unit B for reacting at -78 °C to -20 °C to obtain a reaction solution containing compound ii;
or i'- 1) premixing the solution of compound i and the solution of methylation reagent and then feeding same with the solution of hydrogen-pulling reagent into the unit A or B for reacting at -78 °C to -20 °C to obtain the reaction solution of compound ii;
or i" - 1) premixing the solution of compound i and the solution of methylation reagent in the unit A and then feeding same with the solution of hydrogen-pulling reagent into the unit B for reacting at -78 °C to -20 °C to obtain the reaction solution of compound ii.

4. The method according to claim 2, wherein the post-processing module comprises a reaction unit C, and the step S2) comprises the following steps:
i-3) flowing the obtained reaction solution of compound ii into the unit C, and at the same time feeding an acid solution into the unit C for quenching reaction at -78 °C to -20 °C to obtain a stable quenching reaction solution containing compound ii.

5. The method according to any one of claims 1-4, wherein the solvent of the acid solution is selected from water, alcohol, or a combination thereof, and the acid is selected from hydrochloric acid, sulfuric acid, or a combination thereof.

6. The method according to any one of claims 1-5, wherein the acid solution comprises a salt or no salt, preferably, the salt is NaCl, KCl, NH₄Cl, or a combination thereof.

7. The method according to any one of claims 1-6, wherein the purity of the compound of formula ii obtained in step S2) after post-processing is greater than 90%, preferably greater than 94%, more preferably greater than 95%, more preferably greater than 96%, more preferably greater than 97%, and more preferably greater than 98%.

8. The method according to any one of claims 1-7, wherein the molar ratio of compound i to acid is 1:5.0-10.0.

9. The method according to claim 4, wherein the residence time of the reaction solution in the unit C is not more than 100 seconds.

10. The method according to any one of claims 1-9, wherein the microreactor is composed of one or more of the following reactors: Corning reactor, micro-well (silicon carbide, Hastelloy, 316L) reactor, and Shen's (Hastelloy, 316L) reactor.

## Patentansprüche

1. Verfahren zur kontinuierlichen Synthese der Verbindung nach Formel ii unter Verwendung eines Mikroreaktors, wobei dieses die folgenden Schritte umfasst:
S1) Umsetzen der Verbindung i mit einem wasserstoffziehenden Reagenz und einem Methylierungsreagenz bei -78 °C bis -20 °C in einem inerten Lösungsmittel, um eine Reaktionslösung zu erhalten, welche die Verbindung der Formel ii enthält;
S2) Quenchen der in Schritt S1) erhaltenen Reaktionslösung mit einer Säurelösung bei -20 °C bis 40 °C, um die Verbindung der Formel ii zu erhalten;

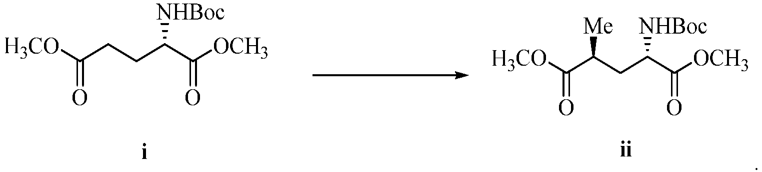

2. Verfahren nach Anspruch 1, wobei der Mikroreaktor ein Reaktionsmodul und ein Nachbearbeitungsmodul umfasst, wobei der Schritt S1) in dem Reaktionsmodul und der Schritt S2) in dem Nachbearbeitungsmodul durchgeführt wird.

3. Verfahren nach Anspruch 2, wobei das Reaktionsmodul eine Reaktionseinheit A und/oder eine Reaktionseinheit B umfasst, und der Schritt S1) die folgenden Schritte umfasst:
i-1) vorkühlen einer Lösung der Verbindung i und einer Lösung eines wasserstoffziehenden Reagenz und anschließendes Einspeisen derselben in die Einheit A zur Reaktion bei -78 °C bis -20 °C;
i-2) die Reaktionslösung aus Schritt i-1) in die Einheit B fließen lassen und anschließendes Einleiten einer Lösung eines Methylierungsreagenz in die Einheit B zur Reaktion bei -78 °C bis -20 °C, um eine Reaktionslösung zu erhalten, die Verbindung ii enthält;
oder i'- 1) vormischen der Lösung von Verbindung i und der Lösung des Methylierungsreagenzes und anschließendes Einspeisen derselben mit der Lösung des wasserstoffziehenden Reagenzes in die Einheit A oder B zur Umsetzung bei -78 °C bis -20 °C, um die Reaktionslösung der Verbindung ii zu erhalten;
oder i"- 1) vormischen der Lösung von Verbindung i und der Lösung des Methylierungsreagenzes in der Einheit A und anschließendes Einspeisen derselben mit der Lösung des wasserstoffziehenden Reagens in die Einheit B zur Umsetzung bei -78 °C bis -20 °C, um die Reaktionslösung der Verbindung ii zu erhalten.

4. Verfahren nach Anspruch 2, wobei das Nachbearbeitungsmodul eine Reaktionseinheit C umfasst, und der Schritt S2) die folgenden Schritte umfasst:
i-3) einfließen lassen der erhaltenen Reaktionslösung von Verbindung ii in die Einheit C und gleichzeitiges Einleiten einer Säurelösung in die Einheit C zur Quenchreaktion bei -78 °C bis -20 °C, um eine stabile Quenchreaktionslösung zu erhalten, die Verbindung ii enthält.

5. Verfahren nach einem der Ansprüche 1 bis 4, wobei das Lösungsmittel der Säurelösung ausgewählt ist aus Wasser, Alkohol oder einer Kombination davon und die Säure ausgewählt ist aus Salzsäure, Schwefelsäure oder einer Kombination davon.

6. Verfahren nach einem der Ansprüche 1 bis 5, wobei die Säurelösung ein Salz oder kein Salz enthält, vorzugsweise ist das Salz NaCl, KCl, NH₄Cl oder eine Kombination davon.

7. Verfahren nach einem der Ansprüche 1 bis 6, wobei die Reinheit der in Schritt S2) erhaltenen Verbindung der Formel ii nach der Nachbehandlung mehr als 90 %, vorzugsweise mehr als 94 %, noch bevorzugter mehr als 95 %, noch bevorzugter mehr als 96 %, noch bevorzugter mehr als 97 % und noch bevorzugter mehr als 98 % beträgt.

8. Verfahren nach einem der Ansprüche 1-7, wobei das molare Verhältnis von Verbindung i zu Säure 1:5,0-10,0 beträgt.

9. Verfahren nach Anspruch 4, wobei die Verweilzeit der Reaktionslösung in der Einheit C nicht mehr als 100 Sekunden beträgt.

10. Verfahren nach einem der Ansprüche 1-9, wobei der Mikroreaktor aus einem oder mehreren der folgenden Reaktoren besteht: Corning-Reaktor, Micro-Well-Reaktor (Siliziumkarbid, Hastelloy, 316L) und Shen's-Reaktor (Hastelloy, 316L).

## Revendications

1. Procédé de synthèse en continu du composé de formule ii en utilisant un microréacteur, comprenant les étapes suivantes:
S1) faire réagir le composé i avec un réactif d'extraction d'hydrogène et un réactif de méthylation à une température de -78 °C à -20 °C dans un solvant inerte pour obtenir une solution réactionnelle contenant le composé de formule ii;
S2) trempage de la solution réactionnelle obtenue dans l'étape S1) avec une solution acide à une température comprise entre -20°C et 40°C, pour obtenir le composé de formule ii;

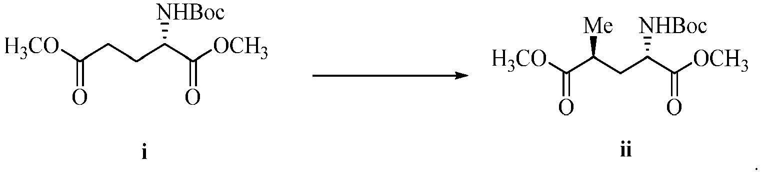

2. Procédé selon la revendication 1, dans lequel le microréacteur comprend un module de réaction et un module de post-traitement, l'étape S1) étant réalisée dans le module de réaction et l'étape S2) étant réalisée dans le module de post-traitement.

3. Procédé selon la revendication 2, dans lequel le module de réaction comprend une unité de réaction A et/ou une unité de réaction B, et l'étape S1) comprend les étapes suivantes:
i-1) prérefroidir une solution du composé i et une solution d'un réactif d'extraction d'hydrogène, puis les introduire dans l'unité A pour qu'elles réagissent entre -78 °C et -20 °C;
i-2) faire s'écouler la solution réactionnelle de l'étape i-1) dans l'unité B, puis introduire une solution d'un réactif de méthylation dans l'unité B pour qu'elle réagisse à une température comprise entre -78 °C et -20 °C afin d'obtenir une solution réactionnelle contenant le composé ii;
ou i'- 1) prémélanger la solution du composé i et la solution du réactif de méthylation, puis l'introduire avec la solution du réactif d'extraction d'hydrogène dans l'unité A ou B pour la faire réagir à une température de -78 °C à -20 °C afin d'obtenir la solution réactionnelle du composé ii;
ou i"- 1) prémélanger la solution du composé i et la solution du réactif de méthylation dans l'unité A, puis l'alimenter avec la solution du réactif d'extraction d'hydrogène dans l'unité B pour une réaction à -78 °C à -20 °C, afin d'obtenir la solution réactionnelle du composé ii.

4. Procédé selon la revendication 2, dans lequel le module de post-traitement comprend une unité de réaction C, et l'étape S2) comprend les étapes suivantes:
i-3) faire couler la solution réactionnelle de composé ii obtenue dans l'unité C et introduire simultanément une solution acide dans l'unité C pour une réaction d'extinction à -78 °C à -20 °C afin d'obtenir une solution réactionnelle d'extinction stable contenant le composé ii.

5. Procédé selon l'une quelconque des revendications 1 à 4, dans lequel le solvant de la solution acide est choisi parmi l'eau, l'alcool ou une combinaison de ceux-ci, et l'acide est choisi parmi l'acide chlorhydrique, l'acide sulfurique ou une combinaison de ceux-ci.

6. Procédé selon l'une quelconque des revendications 1 à 5, dans lequel la solution acide contient un sel ou aucun sel, de préférence le sel est NaCl, KCl, NH₄Cl ou une combinaison de ceux-ci.

7. Procédé selon l'une quelconque des revendications 1 à 6, dans lequel la pureté du composé de formule ii obtenu dans l'étape S2) après post-traitement est supérieure à 90 %, de préférence supérieure à 94 %, plus préférentiellement encore supérieure à 95 %, plus préférentiellement encore supérieure à 96 %, plus préférentiellement encore supérieure à 97 % et plus préférentiellement encore supérieure à 98 %.

8. Procédé selon l'une quelconque des revendications 1 à 7, dans lequel le rapport molaire du composé i à l'acide est de 1:5,0-10,0.

9. Procédé selon la revendication 4, dans lequel le temps de séjour de la solution réactionnelle dans l'unité C n'est pas supérieur à 100 secondes.

10. Procédé selon l'une quelconque des revendications 1 à 9, dans lequel le microréacteur est constitué d'un ou de plusieurs des réacteurs suivants: réacteur Corning, réacteur Micro-Well (carbure de silicium, Hastelloy, 316L) et réacteur Shen's (Hastelloy, 316L).
